# EUROPEAN PATENT SPECIFICATION

(11) **EP 4 514 443 B1**
(45) Date of publication and mention of the grant of the patent: **14.01.2026**
(21) Application number: 23723508.0
(22) Date of filing: 28.04.2023
(51) Int. Cl.: A61N 1/04, A61B 5/256, A61B 5/274, A61B 5/00

(54) **DRY ELECTRODE INTEGRATED WITH GARMENT**
IN EIN KLEIDUNGSSTÜCK INTEGRIERTE TROCKENELEKTRODE
ÉLECTRODE À SEC INTÉGRÉE À UN VÊTEMENT

(30) Priority: 29.04.2022 EP 22170697
(43) Date of publication of application: 05.03.2025
(73) Proprietor: Aarhus Universitet, 8000 Aarhus C (DK); Suetta, Charlotte, 2000 Frederiksberg (DK)
(72) Inventor: MERHI, Yuossif Yaacoub, 8000 Aarhus C (DK); AGARWALA, Shweta, 8000 Aarhus C (DK); MIKKELSEN, Peter Høgh, 8000 Aarhus C (DK); NYGAARD, Jens Vinge, 8000 Aarhus C (DK); SUETTA, Charlotte, 2000 Frederiksberg (DK)
(74) Representative: Plougmann Vingtoft a/s
(86) International application number: PCT/EP2023/061236
(87) International publication number: WO 2023/209148

(56) References cited:
- WO-A1-2020/190405
- WO-A1-2020/201528
- WO-A2-2020/239950
- US-A1- 2021 401 343

## Description

### FIELD OF THE INVENTION

The present invention relates to the field of electrodes for medical purposes, such as for neuro muscular electrical stimulation (NMES) on a person, more specifically to a structure of a dry electrode integrated with a garment, such as a compression garment, and suited for long time contact with the skin of a person.

### BACKGROUND OF THE INVENTION

Electrodes for contact with the skin of a person for performing neuro muscular electrical stimulation (NMES) are well-known, e.g. for performing NMES on the leg of a person.

However, NMES is normally performed over a rather short time, such as for minutes. Thus, electrodes for such NMES are applied before starting NMES therapy and removed again after the NMES therapy.

For patient being immobile, e.g. in a bed at a hospital, for a long period of times, NMES can be used to prevent edema and reduce loss of muscle mass. However, using known electrodes, medical personnel must be involved in mounting the electrodes before NMES therapy and remove the electrodes again after the therapy to avoid skin irriation. This is a tedious process and thus practically limits the number of NMES therapy sessions performed during one day on a person. In this way only a limited effect of the NMES therapy can be expected.

Prior art is provided by US 2021/401343 A1, WO 2020/239950 A2, WO 2020/190405 A1 and WO 2020/201528 A1.

### SUMMARY OF THE INVENTION

Thus, according to the above description, it is an object of the present invention to provide an electro and an NMES system which solves the problem with many manually operations required to operate an NMES system for effective NMES therapy.

In a first aspect, the invention provides a dry electrode integrated with a garment to be worn by a subject, the dry electrode comprising a layered structure comprising
- a first layer comprising a polymeric material, wherein the first layer has a lower side serving to adhere to the garment and an upper side, preferably a plane upper side,
- a second layer of an electrically conductive material, such as comprising a metal, arranged, such as imposed, on the upper side of the first layer, and
- a third layer with a surface serving to be in contact with the skin of the subject when wearing the garment,

wherein the third layer comprises a rubber material, such as silicone, with electrically conductive particles therein, so as to provide a higher electrical impedance than the second layer, and
wherein the third layer is in contact with the second layer and covers an area large enough to cover both of the first and second layers, and so that an edge portion, of the third layer adheres directly to the garment, such as the edge portion having a width of at least 2 mm, such as at least 5 mm, such as 5-10 mm.

Such dry electrode has been found to solve the above mentioned problems with performing efficient NMES on e.g. hospitalized patients over a long period of time. The dry electrode integrated with e.g. a compression stocking can be worn by the patient over a long period without skin irritation, and thus the dry electrode or electrodes are functional for application of many short NMES sessions without any prior step of mounting the electrodes on the skin of the patient. E.g. an automatic NMES stimulation device can be permanently connected to the dry electrode(s) and programmed to perform NMES sessions several times a day, such as both day and night. This allows an efficient NMES therapy without any unnecessary disturbance of patient and with a minimal time spent by medical personnel.

A prototype of the dry electrode has been tested on a hospitalized patient, and a muscle preservation effect has been observed by NMES therapy using the dry electrode.

Still further, the dry electrode can be integrated with a compression garment and has been tested to be durable even by repeated washing of the garment with the dry electrode integrated thereon. This is obtained, since the layered structure of the dry electrode can be made thin and resilient to allow the layered structure to follow stretching of the compression garment without any peeling effects.

In the following, preferred features and embodiments will be mentioned.

The layered structure is preferably resilient or elastic, hereby allowing the layered structure to follow stretches of the garment on which it is integrated. Especially, this may be obtained with the layered structure having a total thickness of such as 0.2-1 mm, such as 0.3-0.7 mm, such as 0.4-0.6 mm.

Preferably, the layered structure is perforated by a plurality of through-going perforation holes to allow perspiration through the dry electrode. Especially, the a pattern of perforation holes may cover at least 50% of a total area covered by the dry electrode, such as at least 80%, such as at least 90% of a total area covered by the dry electrode. In this way, a higher wearing comfort can be obtained, thereby also making long time skin contact possible.

Preferably, the dry electrode comprises an electric connector for external electric contact, such as a snap connector, wherein the electric connector is electrically connected to the second layer. Especially, the dry electrode may comprise a conductive connector part which penetrates at least the the garment and the lower layer, wherein the conductive connector part is in electric contact with the conductive layer, and wherein the conductive connector part is electrically connected to a connector head arranged on an opposite side of the garment.

The third layer is preferably in direct contact with an upper surface of the second layer, preferably the entire upper surface of the second layer, hereby providing a good electric contact between the second and third layers, so as to distribute an electric current applied to the second layer over a large area of the third layer for homogeneous distribution of the electric current to the skin of the subject.

The first layer can be a material comprising at least one of: polyamide polytetrafluoroethylene (PTFE), a polyurethane, a polyethylene, a polypropylene, polyethylene terephelate, and a polyester. In preferred embodiments, the first layer is formed by a polyurethane material. The thickness of the first layer may have a thickness of 0.01-1 mm, preferably 0.05-0.3 mm, such as 0.05-0.15 mm. This has been found as a suitable material and thickness on e.g. compression garment to provide an interface layer for application of the second layer.

The second layer is preferably formed by a metallic material, such as a metallic foil, film or coating, disposed on the upper surface of the first layer (L1).has a thickness of 0.05-0.5 mm, such as 0.1-0.3 mm.

The third layer preferably has an electric DC resistance per square being a factor of at least 10, such as 10-1000, such as at least 100, such as at least 1000, higher than an electric DC resistance per square of the second layer. This has been found as suitable for providing a good electric current distribution to the skin of the subject.

The edge portion of the third layer preferably has a width of of at least 2 mm. More preferably such as at least 3 mm, such as at least 4 mm, such as at least 5 mm, such as 3-10 mm, such as 4-6 mm. Especially, the edge portion may have a width of at least 5 mm, along an entire periphery of the third layer and wherein the entire edge portion adheres directly to the garment. More specifically, the edge portion may have a width of at least 5 mm and encircling a central area occupied by the first and second layers.

The third layer may especially comprise 60-90 percent (w/w) silicone and 10-40 percent (w/w) conductive particles, such as carbon particles, such as graphene particles. More specifically, the third layer may comprise 65-80 percent (w/w) silicone and 20-35 percent (w/w) carbon or graphene, such as the carbon or graphene having an average particle size below 10 microns.

The third layer may cover an area of at least 1 cm², such as at least 10 cm², such as at least 20 cm², such as at least 50 cm², such as at least 100 cm², such as at least 200 cm². The area size depends on the actual application of the dry electrode, whether for NMES of various body parts or for measurements such as ECG measurements or the like.

Especially, it may be preferred that the electrode is designed so that it is mechanically compatible with skin, preferably such that the electrode exhibits a Youngs modulus of such as 0.30-0.50 MPa, more preferably 0.35-0.45 MPa, such as 0.37-0.42 MPa, such as about 0.39 MPa.

Preferably, the second layer has a higher stiffness than the first layer.

In one emebodiment, the first layer is made of a polyurethane material, and wherein the second layer comprises silver. Especially, the second layer is an ink comprising silver and being printed onto a surface of the first layer. Especially, the first layer may have a thickness of 50-200 µm, such as 70-150 µm, such as 80-120 µm, and wherein the second layer may have a thickness of 30-120 µm, such as 40-80 µm. Especially, the layered structure has a stiffness exhibiting a Young's modulus of 0.30-0.50 MPa, such as 0.35-0.45 MPa, such as 0.37-0.42 MPa, and wherein the second layer has a higher stiffness than the first layer.

In a second aspect, the invention provides a compression garment adapted to provide neuromuscular electrical stimulation to a subject when wearing the compression garment, the compression garment comprising at least two dry electrodes integrated with the compression garment, wherein each of the at least two dry electrodes comprises the dry electrode according to the first aspect.

The compression garment may especially comprise a material comprising elastane and/or elastin, or another type of elastic textile or fabric that can serve for compression when worn by a subject.

The compression garment may be one of: a stocking, a legging, a sock, a leg sleeve, and an arm sleeve.

In a specific embodiment, the compression garment comprises a stocking or legging, wherein the two dry electrodes (EL1, EL2) are positioned on the stocking or legging so as to allow neuromuscular electrical stimulation of a person's leg muscles when wearing the stocking or legging, such as the two dry electrodes being positioned at respective positions on the stocking or legging corresponding to respective positions along the person's thigh and/or calf muscle when wearing the stocking or legging. Especially, each of the two dry electrodes may have an area of 10-200 cm² for contact with the skin of the person wearing the stocking or legging, such as an area of 50-200 cm², such as 70-150 cm². More specifically, each of the two dry electrodes may have a rectangular shape, such as a size and shape of the two dry electrodes being identical or the size of the two dry electrodes may differ by less than 50%, such as less than 20%.

In some embodiments, the at least two dry electrodes are shaped and sized to allow electric measurement of one or more electric signals from the skin of the subject wearing the compression garment.

In some embodiments, in each of the at least two dry electrodes, the second layer has a higher stiffness than the first layer, and the first layer has a higher stiffness than the compression garment. This has proven to be a preferred property of the layered structure to provide a flexible electrode which allows the compression garment to be stretched and still allow the layers of the electrode to adhere well to the garment and to provide a good interlayer adhesion.

In a third aspect, the invention provides a neuromuscular electrical stimulation system comprising
- a compression garment according to the second aspect, and
- a stimulation device electrically connected to the at least two dry electrodes and being configured to provide a neuromuscular electrical stimulation pattern to the at least two dry electrodes.

In a fourth, however non-claimed aspect, the disclosure provides a method for manufacturing a dry electrode according the first aspect, the method comprising
- providing a garment,
- depositing, such as by printing or by adhering a sheet onto the garment by means of heating, the first layer onto the garment,
- depositing, such as by printing, the second layer on the upper side of the first layer, and
- depositing, such as by printing, the third layer so as to cover the first and second layers, and so that the edge portion of the third layer adheres directly to the garment.

In a fifth, however non-claimed aspect, the disclosure provides a medical therapy on a living human or animal body comprising applying neuromuscular electrical stimulation to a subject by means of the dry electrode according to the first aspect or the compression garment according to any of the second aspect, such as by applying a series of neuromuscular electrical stimulation sessions of over a period of time.

Especially, the neuromuscular electrical stimulation may serve to prevent or reduce edema and/or to preserve muscle mass of the subject. Especially, the medical therapy may comprise applying a series of neuromuscular electrical stimulation sessions over a period of more than 1 day, such as more than 2 days, such as more than 10 days.

It is appreciated that the described embodiments and features for the mentioned aspects can be intermixed in any way.

### BRIEF DESCRIPTION OF THE FIGURES

The invention will now be described in more detail with regard to the accompanying figures of which
FIG. 1a and 1b illustrate side section view and a top view of a dry electrode embodiment integrated with a garment,
FIG. 2a, 2b, and 2c illustrates 3D views of an exploded and assembled dry electrode embodiment with a snap connector for external electric connection of the electrode,

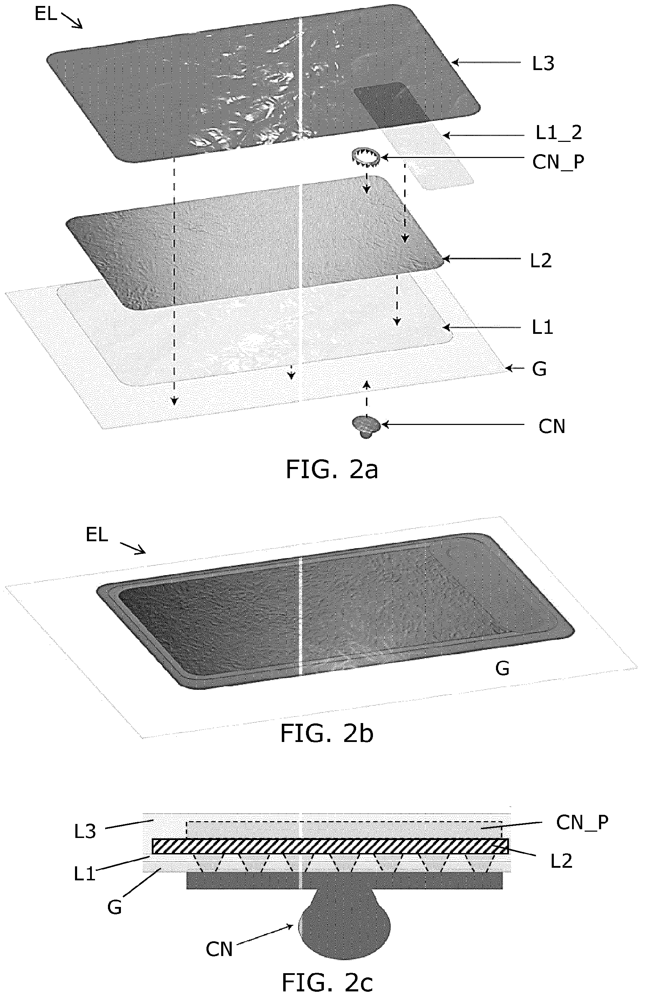

FIG. 2 illustrates a side section view of detail of a dry electrode embodiment showing an example of implementation of an external electric connector for connecting the dry electrode to e.g. an electric stimulation device or an electric measurement device,

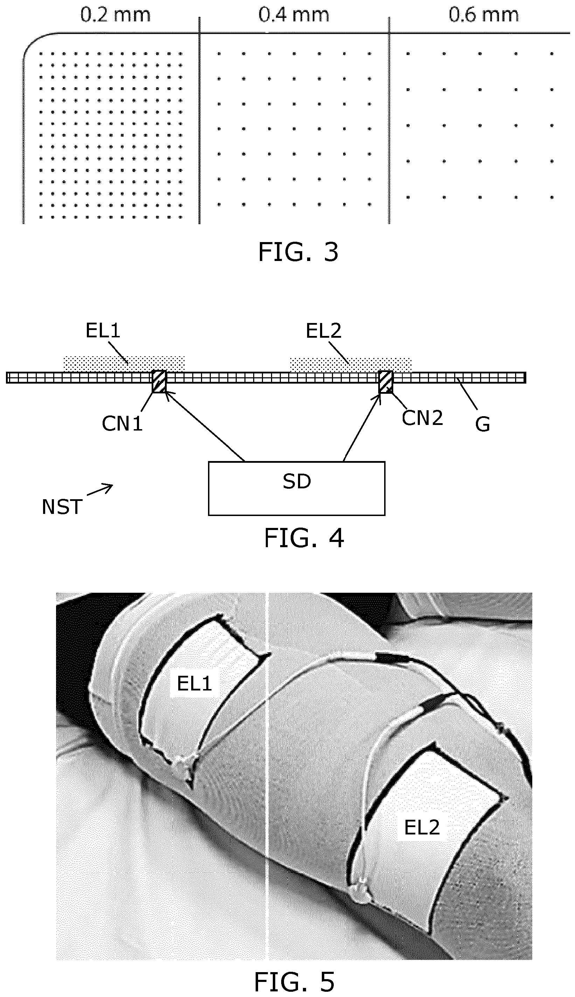

FIG. 3 illustrates examples of patterns of through-going perforations tested to allow perspiration from the skin through the layered structure of the dry electrode,
FIG. 4 illustrates, a system embodiment with a compression garment with two dry electrodes and an NMES stimulation device for generating NMES electric signals to the two dry electrodes,

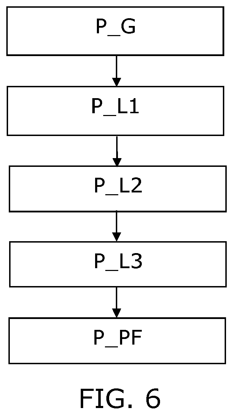

FIG. 5 illustrates a photo of a person wearing a compression stocking with two dry electrode embodiments for NMES stimulation of the person's leg muscles, and FIG. 6 illustrates steps of a method of manufacturing embodiment.

The figures illustrate specific ways of implementing the present invention and are not to be construed as being limiting to other possible embodiments falling within the scope of the attached claim set.

### DETAILED DESCRIPTION OF THE INVENTION

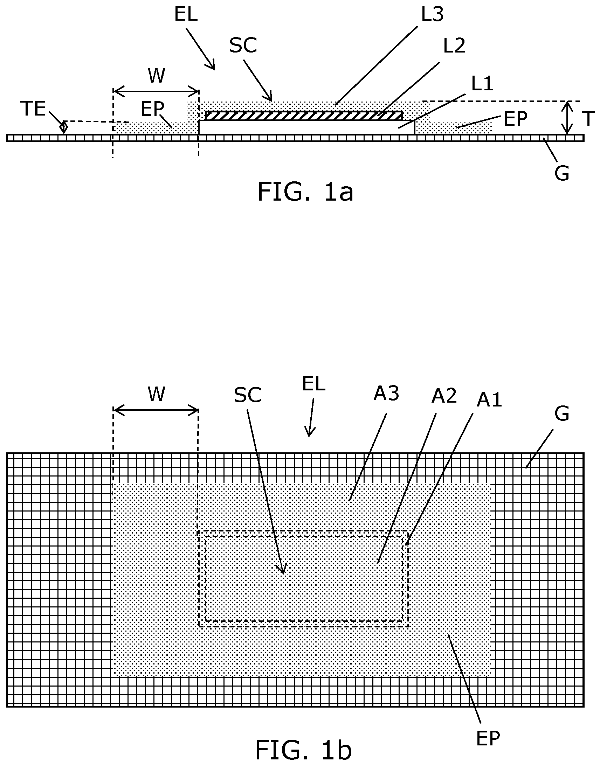

FIG. 1a illustrates a side and FIG. 1b illustrates a top view of a dry electrode EL embodiment integrated with a garment G. The garment G can be a compression garment, such as an elastane based garment, or other textile or the like, which forms a piece of clothes to be worn by a subject (a person or an animal). The dry electrode EL is especially advantageous for application of an electric current to the skin of the subject for electric stimulation or to measure an electric signal on the skin of the subject.

The dry electrode EL is to be used in direct contact with the skin of the subject without any gel or the like. This allows the dry electrode EL to be used integrated in the garment G of a stocking or the like to be worn by the subject for a long time, e.g. one day or more than one day. This is possible, since the selected material for contact with the subject's skin does not cause skin irritation. Furthermore, the electrode EL can be perforated to allow perspiration through the layered structure which further increases the comfort for the subject and thereby allows long-term wearing of the garment G with the electrode EL in permanent contact with the skin.

Hereby, it is possible to have the electrode EL permanently connected to e.g. an NMES stimulation device or to an electro cardiography (ECG) device without the need for medical personnel to mount and take off the electrodes at the time when the electrodes are intended to be in function, e.g. for NMES stimulation or for ECG measurements. This is convenient e.g. for a patient being hospitalized for a longer period, e.g. for frequently applying short session of NMES to prevent loss of muscle mass of the leg muscles. NMES can be automatically applied by an NMES stimulation device connected to the electrode EL and being programmed to apply NMES stimulation may times a day. Even further, the constantly mounted electrode EL allows NMES stimulation on a patient at all times, since there is no electrode mounting procedure involved. In this way effective NMES stimulation is possible, thereby preserving muscle mass and preventing edema for hospitalized patients.

The dry electrode EL comprises a layered structure with three layers L1, L2, L3, as seen clearly in FIG. 1a.

The lower layer L1 serves an interface layer between the garment G and the further layers L2, L3. The lower layer is made of a polymeric material, preferably a polyurethane material, which has been found to adhere well to typical garments G, e.g. by disposing the lower layer L1 in a material printing process or by providing the lower layer L1 as a preprocessed sheet and applying this sheet directly to the garment by heating the material to cause the material to adhere directly to the garment G without the use of any adhesive material. In either way of manufacturing the lower layer L1, the lower layer L1 thus has a lower side which adheres directly to the garment G and a plane or substantially plane upper side which provides a suitable surface for application of the next layer L2.

The lower layer L1 can be selected to have a thickness depending on the garment G on which it should adhere, so that it provides a suitable smooth upper surface for the next layer L2, and so that it is still elastic enough to allow the lower layer L1 to follow any stretching of the garment G without loosing its adhering effect on the garment G. A lower layer L1 of a polyurethane material with a thickness of such as 0.05-0.15 mm, is found to be suitable, e.g. to allow the next layer L2 to be printed directly onto the surface of the lower layer L1. As a specific example of a polyurethane material to be used for the lower layer L1 is a matte finish sheet or printed/painted layer (BFPRINT01, B-FLEX). E.g. provided as a sheet being heated to adhere to the garment.

The next layer is a conductive layer L2, i.e. an electrically conductive material with a low DC resistance, such as a material providing a DC resistance below 10 Ω/square, preferably below 1 Ω/square, preferably below 100mΩ/square. More specifically, for a silver based film used as the condutive layer L2, a DC resistance of such as 10-50 mΩ/square may be seen. This material may comprise a metal or being a metal such as silver or a material comprising silver. The conductive layer L2 can be diposed on the upper side of the lower layer L1 either by material printing or by simply placing a foil or sheet of metal onto the upper side of the lower layer L1. The conductive layer preferably L2 covers an area A2 which is slightly smaller than, or maybe equal to, the area A1 covered by the lower layer L1, see also FIG. 1b, since preferably the conductive layer L2 preferably adheres to the lower layer L1 or at least is applied directly onto the lower layer L1.

The upper layer L3 has a surface SC serving to be in direct contact with the skin of the subject when wearing the garment G and thus provides an electric interface between the conductive layer L2 and the skin of the subject. The upper layer serves as an encapsulation layer L3. The encapsulation layer L3 completely encapsulates the lower layer L1 and the conductive layer L2 together with the garment G. In this way it is ensured that there is no direct contact between the conductive layer L2 and the skin, whereas the conductive layer L2 merely serves to provide an electric current to the upper layer L3, and the upper layer is made of a material which has a higher electrical impedance (e.g. measured as DC resistance per square) than the conductive layer L2, such as a factor of 10-1000 higher. This has been found to provide a good distribution of electric current in the skin of the subject without any discomfort for the subject when an electric current is applied to the conductive layer L2, e.g. for NMES. This allows effective (rather high power) NMES without any discomfort.

The upper layer L3 is preferably made of a silicone with homogeneously distributed electrically conductive particles, such as graphene particles or metallic particles. In this way a suitable electrical conductivity can be provided for the upper layer L3.

The upper layer L3 is in direct contact with the conductive layer L2 and covers an area A3 large enough to cover both of the first and second layers L1, L2, i.e. areas A1 and A2 see also the top view of FIG. 1b. Further, it is seen in FIG. 1a and 1b that the upper layer L3 provides an edge portion EP which adheres directly to the garment G to provide the mentioned encapsulation effect, and which is important for providing a good adhering of the layered structure to the garment G without any peeling effects, e.g. for reusable garments G with the integrated electrode EL that needs to be washed. Most preferably, the edge portion EP has a width W along all of the periphery of the area A3 covered by the upper layer L3 of at least 5 mm, e.g. 5-7 mm. This has been found to be suitable for a good adhering effect without any peeling effects even after several times of washing. The thickness TE of the the edge portion EP of the upper layer L3 material can be different from a thickness of the upper layer L3 where the upper layer L3 covers the conductive layer L2, e.g. the edge portion EP of the upper layer L3 may be thinner than the other part of the upper layer L3. However, the thickness of the upper layer L3 is preferably uniform and such as within 0.1-0.5 mm, more preferably within 0.15-0.30 mm, such as 0.20-0.25 mm, more specifically in a specific embodiment the thickness can be 0.22 mm +/- 5%.

The third layer L3 can be provided by means of screen printing, thus providing a uniform thickness.

The total thickness (or height) T of the electrode EL can be such as 0.4-0.6 mm. With such thickness, the total layered structure of the electrode EL is flexible and elastic enough to follow stretches of the garment G, even in case of thin elastic garments, such as used for compression stockings or the like, e.g. a garment G made of elastane.

Especially, it may be preferred that the electrode EL is designed so that it is mechanically compatible with skin, preferably such that the electrode EL exhibits a Youngs modulus of such as 0.30-0.50 MPa, more preferably 0.35-0.45 MPa, such as about 0.39 MPa.

In FIG. 1b a rectangular shaped electrode EL is illustrated, however it is to be understood that the size and shape of the electrode EL can be designed depending on the application. E.g. whether for NMES applications which can be performed on different smaller or larger body parts or whether for electric measurements. To provide the most durable electrode, e.g. for washing, it may be preferred that the corners are rounded, in case of a rectangular shaped overall design. This can be seen in the following embodiment.

FIG. 2a and 2b illustrates 3D views of an exploded and assembled dry electrode EL embodiment with a metal snap connector CN, CN_P to provide electric connection to the conductive layer L2 of the dry electrode EL. The embodiment of FIG. 2a, 2b has a general rectangular shape with rounded corners.

The layered structure of the electrode EL is positioned on one side of the garment G, while the snap connector head CN with a protrusion for external electric connection providing a snap effect, is positioned on the opposite side of the garment G. As seen, the snap connector head CN made of metal is brought into electric contact with the conductive layer L2 of the electrode EL by a metal connector part CN_P which is arranged between the conductive layer L2 and the upper layer L3 and has a ring with spikes. The spikes penetrate the conductive layer L2, the interface layer L1 and the garment G and forms electric contact with the connector head CN. The spikes of the connector part CN_P may further serve to mechanically attach to the connector head CN, so that the connector head CN is locked in position.

The connector head CN is shown positioned near a corner of the electrode EL. E.g. the connector hear CN may be positioned such as at a distance of 3-10 mm, such as 4-6 mm, from edges of the lower layer L1.

The connector head CN may especially have a diameter of such as 5-10 mm.

In a specific version of the embodiment shown in FIG. 2a, 2b, the lower layer or interface layer L1 is made of polyurethane and has dimensions 130x60 mm with rounded corners with radius of 4-8 mm, such as 6 mm, and a thickness of 0.06-0.12 mm, more preferably a thickness of 0.08-0.10 mm. The conductive layer L2 is made of a metal, such as silver, and has dimensions 125x55 mm, thus covering about 88% of the lower layer L1, and having a thickness of 0.10-0.20 mm, such as 0.15 mm or about 0.15 mm. The upper layer or encapsulation layer L3 is made of a silicone with carbon particles distributed therein, and has dimensions 140x70 mm with rounded corners with corner radius similar to the corner radius of the interface layer L1, so as to provide an edge portion of 5 mm or about 5 mm along the entire periphery of the encapsulation layer L3. Thus, the upper layer or encapsulation layer L3 covers an area of about 125% of the area covered by the lower layer or interface layer L1. The upper layer or encapsulation layer L3 preferably has a thickness of 0.15-0.30 mm, such as 0.20-0.24 mm, such as about 0.22 mm. In such specific version, the the textile forming the garment G is an elastic compression textile, e.g. comprising elastane, which has a thickness of 0.10-0.20 mm, such as 0.14 mm or about 0.14 mm. The layered structure with the three layers L1, L2, L3 preferably has a total thickness of 0.40-0.50 mm.

As seen in Fig. 2a, an additional interface layer L1_2 for protecting the connector CN, CN_P and strengthening the layered structure in the area of the connector CN, CN_P. The additional interface layer L1_2 is preferably made of a polymeric material, e.g. the same polyurethane material as the lower layer L1. The additional interface layer L1_2 is added between the connector part CN_P and the upper layer L3. In a specific embodiment, the additional interface layer L1_2 has dimensions 20x60 mm with a material and thickness similar to the lower layer L1. Thus, with this additional interface layer L1_2, the total thickness of the electrode EL is about 0.50-0.60.

It is to be understood that the dimensions of the electrode EL can be selected to suit the application, e.g. the body part intended to receive NMES via the electrode EL. Especially, it may be preferred that two NMES electrodes, an upper and a lower electrode, for a compression stocking have different size. Especially, the upper electrode may be slightly smaller than the lower electrode, such as the upper electrode having an area 5-20% smaller than the lower electrode. Specifically, the lower electrode may have an upper layer L3 of 160x70 mm, whereas the upper electrode may have an upper layer L3 of 140x70 mm.

FIG. 2c shows a side section view of a detail of the layered structure G, L1, L2, L3 electrode EL where the connector head CN is positioned. The position of the connector part CN_P is indicated with dashed line, i.e. both the element positioned on the upper side of the conductive layer L2 and the penetrating spikes that penetrate through the conductive layer, the interface layer L1 and the garment G for contacting the connector head CN.

A protruding part of the connector head CN is seen, and it serves to allow snap on of a corresponding electric connector.

FIG. 3 illustrates examples of patterns of through-going perforations tested to allow perspiration from the skin through the layered structure of the dry electrode. Such perforations can increase wearing comfort and allows the dry electrode to be used for a long period without being removed from the skin.

FIG. 3 show regular patterns of perforations with three different two-dimensional mutual distances between the perforation holes of 0.2 mm, 0.4 mm and 0.6 mm. However, a preferred range of such mutual distances between the perforation holes may be such as 0.1-2.0 mm.

It is to be understood that the actual perforation pattern is not important, rather that perforations are distributed over the majority of the total area covered by the dry electrode. Preferably, the perforations may cover at least 50% of a total area covered by the dry electrode, more preferably at least 80%, such as at leat 90%, of the total area covered by the dry electrode. The preferred size of the holes is such as 1-100 µm, such as 5-50 µm, such as 2-20 µm, and this can be provided e.g. by means of laser cutting. The preferred size of the holes may be depend on the structure of the garment on which the dry electrode is integrated.

As an alternative to, or in addition to, perforations to allow perspiration through the dry electrode, the layered structure may have a plurality of openings to allow natural ventilation through the layered structure, such as openings each having an area of at least 1 mm², such as an area of 1-100 mm².

FIG. 4 illustrates an NMES system embodiment with a compression garment G on which two dry electrodes EL1, EL2 are integrated with a mutual distance. An electric stimulation device SD is electrically connected to the at least two dry electrodes EL1, EL2 and being configured to provide a neuromuscular electrical stimulation pattern to the at least two dry electrodes EL1, EL2.

E.g. for hospitalized patients, the electric stimulation device SD may be connected to the electrodes EL1, EL2 when the patient is in bed and wearing the compression garment G. The electric stimulation device SD can then be programmed to provide NMES to the patient at regular or irregular intervals during the day, or even at night when the patient is sleeping. Hereby effective NMES can be applied to prevent edema and preserve muscle mass.

FIG. 5 illustrates a photo of a person wearing a compression stocking embodiment with two dry electrodes EL1, EL2 positioned on the thigh and/or calf muscle of the person. The electrodes are positioned with a mutual distance of such as 15-20 cm on the garment of the stocking. As seen, electric wires are connected to snap connectors on the dry electrodes EL1, EL2 so as to allow application of an electric signal to the dry electrodes EL1, EL2 to allow NMES to the leg musle of the person. The compression stocking has been tested on a patient wearing the stocking on one leg for a longer period of hospitalization and having performed NMES therapy sessions multiple times a day. A muscle preservation effect was observed when compared to the patient's other leg which did not receive any NMES therapy.

FIG. 6 illustrates steps of a method of manufacturing embodiment. The method comprises providing P_G a garment G, such as a compression garment. Next, depositing P_L1 the first layer L1 onto the garment G. This can be done by material printing or by adhering a sheet onto the garment by means of heating. Next, depositing P_L2 the second layer L2 on the upper side of the first layer L1. This can be done by material printing, or by placing an electrically conducting foil onto the first layer. Next, depositing P_L3 the third layer L3 so as to cover the first and second layers L1, L2, and so that the edge portion of the third layer L3 adheres directly to the garment G. This can be done by material printing, or by applying a sheet of material and provide heating so as to allow the silicone based material to adhere to the garment along the edge portion. Especially, the third layer L3 may be applied in a screen printing process. Next, after the layered structure has been manufactured, a preferred step of performing a perforation P_PF of the layered structure to provide a through-going perforation with a pattern of small holes, e.g. 10-100 µm, that can allow perspiration through the layered structure. This can be performed e.g. by means of laser cutting or by means of hole punching. Preferably, a pattern covering the majority of the total area covered by the dry electrode can be perforated with a perforation pattern having a mutual distance between the holes of such as 0.1-1.0 mm.

In the following, a decription of a specific embodiment of an electrode integrated on an anti-embolism compression stocking will be given with respect to the textile and the two lower layers of the electrode. The stocking was made of Spandex (elastane), nylon, and silicone/polyisoprene (strap) manufactured by CAROLYN and purchased from Mediq (Denmark). The first layer (buffer layer or interface layer) of the electrode (B-FLEX BF PRINT00-BF PRINT01) was made of PU/Polyurethane purchased from Vikiallo (Denmark). The second layer (conductive layer) was made of biocompatible thermally curable silver ink (Fabink, TC-4007) purchased from Smart Fabric Inks Ltd (Southampton, UK). Further chemicals were purchased from Sigma Aldrich. The dimention of the electrode was 120x50 mm.

The interface layer was precut using a desktop cutter and laminated on the textile of the stocking by heat pressing using a manual heat presser. The heat and pressure makes the interface layer adhere well to the textile, and the resulting interface layer provided a smooth surface. The interface layer had a thickness of about 100 µm, and the interface layer was measured to have a roughness R_{q} of 0.27 µm, while the roughness of the stocking textile was measured to be more than 150 µm.

The conductive layer was screen printed on top of the smooth surface of the interface layer and processed at 100°C for 10 minutes in hot air for curing. The printed silver ink layer had a thickness of about 60 µm. The conductive layer was measured to have a roughness R_{q} of 0.30 µm. Further, electric connectors were added.

The third layer (upper layer) of the electrode was added as already described in the foregoing.

Mechanical tests were performed on: 1) the stocking textile, 2) the stocking textile with the interface layer applied, and 3) the stocking textile with both the interface layer and the printed silver ink layer applied. These tests show that all three materials show a relaxation over similar time scale at about 0.8 s. The stiffness of the textile is lowest, the interface layer has a higher stiffness than the texile, while the silver ink layer has a higher stiffness than the interface layer. This progression in stiffness of the layers has been found to be advantageous since it factilitates a structural graduation across the layers and ensure compatibility and improved interlayer adjesion properties when the combined lamina is stretched.

The electrode embodiment with the two just described layers has been tested with repect to its ability to withstand repeated washing in a commercial washing machine. 5 washes were performed, each wash had a duration of 30 minutes and a water temperature of 40°C. The electric resistance was observed after each wash, and the electric resistance increased after each wash, starting at about 5 Ω and ended above 20 Ω after 5 washes. This indicates, that at least the electrode can withstand a few washes, but after a number of washes, the electrode can be expected to be electrically non-functional.

Skin-electrode impedance measurements indicated that the proposed dry electrode has a better signal transmission than commercial gel-based electrode.

In a pilot study with the just described stocking and electrode embodiment, seven men completed five days of treatment with no complications or skin, and it is to be noted that the stocking and electrode was warn during the entire hospital stay. The stocking and electrode was used for NMES on the thigh of one leg, while the opposite leg served as control. The NMES stimulation protocol was 60 Hz/400 µs, 30 minutes two times a day. The thickness of the vastus lateralis was measured before and after the five days using a Doppler ultrasound system. The five days of hospitalization resulted in a muscle mass loss of 8.4% on the control leg compared to the NMES treated leg.

To sum up, the invention provides a dry electrode (EL) integrated with a garment (G) to be worn by a subject, e.g. a compression stocking. The dry electrode (EL) is formed by a layered structure with at least three layers. A first layer (L1) of a polyurethane material with a lower side serving to adhere to the garment (G). A second layer (L2) of an electrically conductive material is arranged on, e.g. printed on, an upper side of the first layer. A third layer (L3) has a surface (SC) serving to be in contact with the skin of the subject when wearing the garment (G). The third layer (L3) is made of a silicone with electrically conductive particles therein, e.g. carbon particles, so as to provide a higher electrical impedance than the second layer (L2). The third layer (L3) is in contact with the second layer (L2) and covers an area (A3) large enough to cover both of the first and second layers (L1, L2), and so that an edge portion (EP), of the third layer (L3) adheres directly to the garment (G), such as the edge portion (EP) having a width of at least 5 mm. The dry electrode is suitable for neuromuscular electrical stimulation (NMES), and it can be worn over a long period of time without skin irritation, e.g. for NMES to preserve muscle mass for hospitalized patients.

Although the present invention has been described in connection with the specified embodiments, it should not be construed as being in any way limited to the presented examples. The scope of the present invention is to be interpreted in the light of the accompanying claim set. In the context of the claims, the terms "including" or "includes" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

## Claims

1. A dry electrode (EL) integrated with a garment (G) to be worn by a subject, the dry electrode (EL) comprising a layered structure comprising
- a first layer (L1) comprising a polymeric material, wherein the first layer (L1) has a lower side serving to adhere to the garment (G) and an upper side, preferably a plane upper side,
- a second layer (L2) of an electrically conductive material, such as comprising a metal, arranged, such as imposed, on the upper side of the first layer (L1), and
- a third layer (L3) with a surface (SC) serving to be in contact with the skin of the subject when wearing the garment (G),
wherein the third layer (L3) comprises a rubber material, such as silicone, with electrically conductive particles therein, so as to provide a higher electrical impedance than the second layer (L2), and
wherein the third layer (L3) is in contact with the second layer (L2) and covers an area (A3) large enough to cover both of the first and second layers (L1, L2), and so that an edge portion (EP), of the third layer (L3) adheres directly to the garment (G), such as the edge portion (EP) having a width of at least 2 mm, such as at least 5 mm, such as 5-10 mm.

2. The dry electrode (EL) according to claim 1, wherein the layered structure is resilient, such as the layered structure being elastic and having a total thickness (T) of 0.2-1 mm, such as 0.3-0.7 mm, such as 0.4-0.6 mm.

3. The dry electrode (EL) according to any of the preceding claims, wherein the layered structure is perforated by a plurality of through-going perforation holes to allow perspiration through the dry electrode EL.

4. The dry electrode (EL) according to any of the preceding claims, comprising an electric connector (CN) for external electric contact, such as a snap connector (CN), wherein the electric connector (CN) is electrically connected to the second layer (L2).

5. The dry electrode (EL) according to any of the preceding claims, wherein the third layer (L3) is in direct contact with an upper surface, such as an entire upper surface, of the second layer (L2).

6. The dry electrode (EL) according to any of the preceding claims, wherein the first layer (L1) is formed by a polyurethane material and having a thickness of 0.01-1 mm, preferably 0.05-0.3 mm, such as 0.05-0.15 mm.

7. The dry electrode (EL) according to any of the preceding claims, wherein the second layer (L2) is formed by a metallic material, such as a metallic foil, film or coating, disposed on the upper surface of the first layer (L1), such as having a thickness of 0.05-0.5 mm, such as 0.1-0.3 mm.

8. The dry electrode (EL) according to any of the preceding claims, wherein the third layer (L3) has an electric DC resistance per square being a factor of at least 10, such as 10-1000, such as at least 100, such as at least 1000, higher than an electric DC resistance per square of the second layer (L2).

9. The dry electrode (EL) according to any of the preceding claims, wherein the edge portion (EP) of the third layer (L3) has a width of (W) of at least 2 mm, such as at least 3 mm, such as at least 4 mm, such as at least 5 mm, such as 3-10 mm, such as 4-6 mm, such as the edge portion (EP) having a width (W) of at least 2 mm, such as at least 5 mm, along an entire periphery of the third layer (L3) and wherein the entire edge portion (EP) adheres directly to the garment (G), such as the edge portion (EP) having a width (W) of at least 2 mm, such as at least 5 mm and encircling a central area (A1, A2) occupied by the first and second layers (L1, L2).

10. The dry electrode (EL) according to any of the preceding claims, wherein the third layer (L3) comprises 60-90 percent (w/w) silicone and 10-40 percent (w/w) conductive particles, such as carbon particles, such as graphene particles, such as the third layer comprising 65-80 percent silicone and 20-35 percent carbon or graphene, such as the carbon or graphene having an average particle size below 10 microns.

11. The dry electrode (EL) according to any of the preceding claims, wherein the third layer (L3) covers an area (A3) of at least 1 cm², such as at least 10 cm², such as at least 20 cm², such as at least 50 cm², such as at least 100 cm², such as at least 200 cm².

12. The dry electrode (EL) according to any of the preceding claims, wherein the layered structure (L1, L2, L3) has a stiffness exhibiting a Young's modulus of 0.30-0.50 MPa, such as 0.35-0.45 MPa, such as 0.37-0.42 MPa.

13. The dry electrode (EL) according to any of the preceding claims, wherein the second layer (L2) has a higher stiffness than the first layer (L1).

14. The dry electrode (EL) according to any of the preceding claims, wherein the first layer (L1) is made of a polyurethane material, and wherein the second layer (L2) comprises silver, such as the second layer (L2) being an ink comprising silver and being printed onto a surface of the first layer (L1).

15. The dry electrode (EL) according to claim 14, wherein the first layer (L1) has a thickness of 50-200 µm, such as 70-150 µm, such as 80-120 µm, and wherein the second layer (L2) has a thickness of 30-120 µm, such as 40-80 µm.

16. The dry electrode (EL) according to claim 15, wherein the layered structure (L1, L2, L3) has a stiffness exhibiting a Young's modulus of 0.30-0.50 MPa, such as 0.35-0.45 MPa, such as 0.37-0.42 MPa, and wherein the second layer (L2) has a higher stiffness than the first layer (L1).

17. A compression garment (G) adapted to provide neuromuscular electrical stimulation to a subject when wearing the compression garment, the compression garment (G) comprising at least two dry electrodes (EL1, EL2) integrated with the compression garment (G), wherein each of the at least two dry electrodes (EL1, EL2) comprises the dry electrode (EL) according to any of claims 1-16.

18. A neuromuscular electrical stimulation system (NST) comprising
- a compression garment (G) according to claim 17, and
- a stimulation device (SD) electrically connected to the at least two dry electrodes (EL1, EL2) and being configured to provide a neuromuscular electrical stimulation pattern to the at least two dry electrodes (EL1, EL2).

## Patentansprüche

1. Trockenelektrode (EL), die in ein Kleidungsstück (G) integriert ist, das durch ein Subjekt zu tragen ist, wobei die Trockenelektrode (EL) eine Schichtstruktur umfasst, umfassend
- eine erste Schicht (L1), die ein Polymermaterial umfasst, wobei die erste Schicht (L1) eine Unterseite, die dazu dient, mit dem Kleidungsstück (G) zu verkleben, und eine Oberseite, bevorzugt eine ebene Oberseite, aufweist,
- eine zweite Schicht (L2) aus einem elektrisch leitfähigen Material, wie etwa umfassend ein Metall, die auf der Oberseite der ersten Schicht (L1) angeordnet ist, wie etwa darauf aufgebracht ist, und
- eine dritte Schicht (L3) mit einer Fläche (SC), die dazu dient, beim Tragen des Kleidungsstücks (G) mit der Haut des Subjekts in Kontakt zu sein,
wobei die dritte Schicht (L3) ein Gummimaterial, wie etwa Silikon, mit elektrisch leitfähigen Partikeln darin umfasst, um eine höhere elektrische Impedanz als die zweite Schicht (L2) bereitzustellen, und
wobei die dritte Schicht (L3) mit der zweiten Schicht (L2) in Kontakt ist und einen Bereich (A3) abdeckt, der groß genug ist, um sowohl die erste als auch die zweite Schicht (L1, L2) abzudecken, und so, dass ein Kantenabschnitt (EP) der dritten Schicht (L3) direkt an dem Kleidungsstück (G) haftet, wie etwa, dass der Kantenabschnitt (EP) eine Breite von mindestens 2 mm, wie etwa mindestens 5 mm, wie etwa 5-10 mm, aufweist.

2. Trockenelektrode (EL) nach Anspruch 1, wobei die Schichtstruktur nachgiebig ist, wie etwa, dass die Schichtstruktur elastisch ist und eine Gesamtdicke (T) von 0,2-1 mm, wie etwa 0,3-0,7 mm, wie etwa 0,4-0,6 mm, aufweist.

3. Trockenelektrode (EL) nach einem der vorstehenden Ansprüche, wobei die Schichtstruktur durch eine Vielzahl von durchgehenden Perforationslöchern perforiert ist, um Perspiration durch die Trockenelektrode EL hindurch zu ermöglichen.

4. Trockenelektrode (EL) nach einem der vorstehenden Ansprüche, umfassend einen elektrischen Verbinder (CN) für einen externen elektrischen Kontakt, wie etwa einem Schnappverbinder (CN), wobei der elektrische Verbinder (CN) mit der zweiten Schicht (L2) elektrisch verbunden ist.

5. Trockenelektrode (EL) nach einem der vorstehenden Ansprüche, wobei die dritte Schicht (L3) in direktem Kontakt mit einer oberen Fläche, wie etwa einer gesamten oberen Fläche, der zweiten Schicht (L2) ist.

6. Trockenelektrode (EL) nach einem der vorstehenden Ansprüche, wobei die erste Schicht (L1) aus einem Polyurethanmaterial gebildet ist und eine Dicke von 0,01-1 mm, bevorzugt 0,05-0,3 mm, wie etwa 0,05-0,15 mm, aufweist.

7. Trockenelektrode (EL) nach einem der vorstehenden Ansprüche, wobei die zweite Schicht (L2) durch ein metallisches Material gebildet ist, wie etwa eine Metallfolie, einen Film oder eine Beschichtung, das auf der oberen Fläche der ersten Schicht (L1) angeordnet ist, wie etwa eine Dicke von 0,05-0,5 mm, wie etwa 0,1-0,3 mm, aufweist.

8. Trockenelektrode (EL) nach einem der vorstehenden Ansprüche, wobei die dritte Schicht (L3) einen elektrischen Gleichstromwiderstand pro Quadrat aufweist, der um mindestens einen Faktor 10, wie etwa 10-1000, wie etwa mindestens 100, wie etwa mindestens 1000 höher ist als ein elektrischer Gleichstromwiderstand pro Quadrat der zweiten Schicht (L2).

9. Trockenelektrode (EL) nach einem der vorstehenden Ansprüche, wobei der Kantenabschnitt (EP) der dritten Schicht (L3) eine Breite (W) von mindestens 2 mm, wie etwa mindestens 3 mm, wie etwa mindestens 4 mm, wie etwa mindestens 5 mm, wie etwa 3-10 mm, wie etwa 4-6 mm, aufweist, wie etwa der Kantenabschnitt (EP) eine Breite (W) von mindestens 2 mm, wie etwa mindestens 5 mm, entlang des gesamten Rands der dritten Schicht (L3) aufweist und wobei der gesamte Kantenabschnitt (EP) direkt an dem Kleidungsstück (G) haftet, wie etwa der Kantenabschnitt (EP) eine Breite (W) von mindestens 2 mm, wie etwa mindestens 5 mm, aufweist und einen zentralen Bereich (A1, A2) einkreist, der durch die erste und die zweite Schicht (L1, L2) eingenommen wird.

10. Trockenelektrode (EL) nach einem der vorstehenden Ansprüche, wobei die dritte Schicht (L3) 60-90 Gew.-Prozent Silikon und 10-40 Gew.-Prozent leitfähige Partikel, wie etwa Kohlenstoffpartikel, wie etwa Graphenpartikel, umfasst, wie etwa die dritte Schicht 65-80 Prozent Silikon und 20-35 Prozent Kohlenstoff oder Graphen umfasst, wie etwa der Kohlenstoff oder das Graphen eine durchschnittliche Partikelgröße unterhalb von 10 Mikrometern aufweist.

11. Trockenelektrode (EL) nach einem der vorstehenden Ansprüche, wobei die dritte Schicht (L3) einen Bereich (A3) von mindestens 1 cm², wie etwa mindestens 10 cm², wie etwa mindestens 20 cm², wie etwa mindestens 50 cm², wie etwa mindestens 100 cm², wie etwa mindestens 200 cm² abdeckt.

12. Trockenelektrode (EL) nach einem der vorstehenden Ansprüche, wobei die Schichtstruktur (L1, L2, L3) eine Steifigkeit aufweist, die einen Elastizitätsmodul von 0,30-0,50 MPa, wie etwa 0,35-0,45 MPa, wie etwa 0,37-0,42 MPa, zeigt.

13. Trockenelektrode (EL) nach einem der vorstehenden Ansprüche, wobei die zweite Schicht (L2) eine höhere Steifigkeit als die erste Schicht (L1) aufweist.

14. Trockenelektrode (EL) nach einem der vorstehenden Ansprüche, wobei die erste Schicht (L1) aus einem Polyurethanmaterial hergestellt ist und wobei die zweite Schicht (L2) Silber umfasst, wie etwa, dass die zweite Schicht (L2) eine Tinte ist, die Silber umfasst und auf eine Fläche der ersten Schicht (L1) gedruckt ist.

15. Trockenelektrode (EL) nach Anspruch 14, wobei die erste Schicht (L1) eine Dicke von 50-200 µm, wie etwa 70-150 µm, wie etwa 80-120 µm, aufweist und wobei die zweite Schicht (L2) eine Dicke von 30-120 µm, wie etwa 40-80 µm, aufweist.

16. Trockenelektrode (EL) nach Anspruch 15, wobei die Schichtstruktur (L1, L2, L3) eine Steifigkeit aufweist, die einen Elastizitätsmodul von 0,30-0,50 MPa, wie etwa 0,35-0,45 MPa, wie etwa 0,37-0,42 MPa, zeigt und wobei die zweite Schicht (L2) eine höhere Steifigkeit als die erste Schicht (L1) aufweist.

17. Kompressionskleidungsstück (G), das dazu eingerichtet ist, einem Subjekt beim Tragen des Kompressionskleidungsstücks eine neuromuskuläre Elektrostimulation bereitzustellen, wobei das Kompressionskleidungsstück (G) mindestens zwei Trockenelektroden (EL1, EL2) umfasst, die in das Kompressionskleidungsstück (G) integriert sind, wobei jede der mindestens zwei Trockenelektroden (EL1, EL2) die Trockenelektrode (EL) nach einem der Ansprüche 1-16 umfasst.

18. Neuromuskuläres Elektrostimulationssystem (NST), umfassend
- ein Kompressionskleidungsstück (G) nach Anspruch 17 und
- eine Stimulationsvorrichtung (SD), die mit den mindestens zwei Trockenelektroden (EL1, EL2) elektrisch verbunden ist und dazu konfiguriert ist, ein neuromuskuläres elektrisches Stimulationsmuster an die mindestens zwei Trockenelektroden (EL1, EL2) bereitzustellen.

## Revendications

1. Électrode à sec (EL) intégrée à un vêtement (G) destiné à être porté par un sujet, l'électrode à sec (EL) comprenant une structure en couches comprenant
- une première couche (L1) comprenant un matériau polymère, dans laquelle la première couche (L1) présente un côté inférieur servant à adhérer au vêtement (G) et un côté supérieur, de préférence un côté supérieur plat,
- une deuxième couche (L2) d'un matériau électroconducteur, par exemple comprenant un métal, disposé, par exemple imposé, sur le côté supérieur de la première couche (L1), et
- une troisième couche (L3) avec une surface (SC) servant à être en contact avec la peau du sujet lors du port du vêtement (G),
dans laquelle la troisième couche (L3) comprend un matériau en caoutchouc, par exemple du silicone, avec des particules électroconductrices à l'intérieur d'elle, de manière à fournir une impédance électrique supérieure à la deuxième couche (L2), et
dans laquelle la troisième couche (L3) est en contact avec la deuxième couche (L2) et recouvre une zone (A3) suffisamment grande pour couvrir à la fois la première et la deuxième couche (L1, L2), et de sorte qu'une partie de bord (EP) de la troisième couche (L3) adhère directement au vêtement (G), par exemple la partie de bord (EP) présentant une largeur d'au moins 2 mm, par exemple au moins 5 mm, par exemple de 5 à 10 mm.

2. Électrode à sec (EL) selon la revendication 1, dans laquelle la structure en couches est résiliente, la structure en couches étant par exemple élastique et présentant une épaisseur totale (T) de 0,2 à 1 mm, par exemple de 0,3 à 0,7 mm, par exemple de 0,4 à 0,6 mm.

3. Électrode à sec (EL) selon l'une quelconque des revendications précédentes, dans laquelle la structure en couches est perforée par une pluralité de trous de perforation traversants pour permettre la transpiration à travers l'électrode à sec EL.

4. Électrode à sec (EL) selon l'une quelconque des revendications précédentes, comprenant un connecteur électrique (CN) pour un contact électrique externe, par exemple un connecteur à encliquetage (CN), dans laquelle le connecteur électrique (CN) est connecté électriquement à la deuxième couche (L2).

5. Électrode à sec (EL) selon l'une quelconque des revendications précédentes, dans laquelle la troisième couche (L3) est en contact direct avec une surface supérieure, par exemple la surface supérieure entière, de la deuxième couche (L2).

6. Électrode à sec (EL) selon l'une quelconque des revendications précédentes, dans laquelle la première couche (L1) est formée par un matériau en polyuréthane et présentant une épaisseur de 0,01 à 1 mm, de préférence de 0,05 à 0,3 mm, par exemple de 0,05 à 0,15 mm.

7. Électrode à sec (EL) selon l'une quelconque des revendications précédentes, dans laquelle la deuxième couche (L2) est formée par un matériau métallique, tel qu'une feuille, un film ou un revêtement métallique, disposé sur la surface supérieure de la première couche (L1), par exemple présentant une épaisseur de 0,05 à 0,5 mm, par exemple de 0,1 à 0,3 mm.

8. Électrode à sec (EL) selon l'une quelconque des revendications précédentes, dans laquelle la troisième couche (L3) présente une résistance électrique CC qui est, par carré, supérieure à une résistance électrique CC par carré de la deuxième couche (L2) d'un facteur d'au moins 10, par exemple de 10 à 1000, par exemple d'au moins 100, par exemple d'au moins 1000.

9. Électrode à sec (EL) selon l'une quelconque des revendications précédentes, dans laquelle la partie de bord (EP) de la troisième couche (L3) présente une largeur de (W) d'au moins 2 mm, par exemple d'au moins 3 mm, par exemple d'au moins 4 mm, par exemple d'au moins 5 mm, par exemple de 3 à 10 mm, par exemple de 4 à 6 mm, la partie de bord (EP) présentant par exemple une largeur (W) d'au moins 2 mm, par exemple d'au moins 5 mm, sur l'entièreté d'une périphérie de la troisième couche (L3) et dans laquelle l'ensemble de la partie de bord (EP) adhère directement au vêtement (G), la partie de bord (EP) présentant par exemple une largeur (W) d'au moins 2 mm, par exemple d'au moins 5 mm et entourant une zone centrale (A1, A2) occupée par les première et deuxième couches (L1, L2).

10. Électrode à sec (EL) selon l'une quelconque des revendications précédentes, dans laquelle la troisième couche (L3) comprend 60 à 90 % (p/p) de silicone et 10 à 40 % (p/p) de particules conductrices, par exemple des particules de carbone, par exemple des particules de graphène, la troisième couche comprenant par exemple 65 à 80 % de silicone et 20 à 35 % de carbone ou de graphène, par exemple le carbone ou graphène présentant une taille de particule moyenne inférieure à 10 microns.

11. Électrode à sec (EL) selon l'une quelconque des revendications précédentes, dans laquelle la troisième couche (L3) recouvre une zone (A3) d'au moins 1 cm², par exemple d'au moins 10 cm², par exemple d'au moins 20 cm², par exemple d'au moins 50 cm², par exemple d'au moins 100 cm², par exemple d'au moins 200 cm².

12. Électrode à sec (EL) selon l'une quelconque des revendications précédentes, dans laquelle la structure en couches (L1, L2, L3) présente une rigidité montrant un module de Young de 0,30 à 0,50 MPa, par exemple de 0,35 à 0,45 MPa, par exemple de 0,37 0,42 MPa.

13. Électrode à sec (EL) selon l'une quelconque des revendications précédentes, dans laquelle la deuxième couche (L2) présente une rigidité supérieure à la première couche (L1).

14. Électrode à sec (EL) selon l'une quelconque des revendications précédentes, dans laquelle la première couche (L1) est en matériau polyuréthane, et dans laquelle la deuxième couche (L2) comprend de l'argent, par exemple la deuxième couche (L2) étant une encre comprenant de l'argent et étant imprimée sur une surface de la première couche (L1).

15. Électrode à sec (EL) selon la revendication 14, dans laquelle la première couche (L1) présente une épaisseur de 50 à 200 µm, par exemple de 70 à 150 µm, par exemple de 80 à 120 µm, et dans laquelle la deuxième couche (L2) présente une épaisseur de 30 à 120 µm, par exemple de 40 à 80 µm.

16. Électrode à sec (EL) selon la revendication 15, dans laquelle la structure en couches (L1, L2, L3) présente une rigidité montrant un module de Young de 0,30 à 0,50 MPa, par exemple de 0,35 à 0,45 MPa, par exemple de 0,37 à 0,42 MPa, et dans laquelle la deuxième couche (L2) présente une rigidité supérieure à la première couche (L1).

17. Vêtement de compression (G) adapté pour fournir une stimulation électrique neuromusculaire à un sujet lorsqu'il porte le vêtement de compression, le vêtement de compression (G) comprenant au moins deux électrodes à sec (EL1, EL2) intégrées au vêtement de compression (G), dans lequel chacune des au moins deux électrodes à sec (EL1, EL2) comprend l'électrode à sec (EL) selon l'une quelconque des revendications 1 à 16.

18. Système de stimulation électrique neuromusculaire (SNM) comprenant
- un vêtement de compression (G) selon la revendication 17, et
- un dispositif de stimulation (SD) électriquement connecté auxdites au moins deux électrodes à sec (EL1, EL2) et étant configuré pour fournir un schéma de stimulation électrique neuromusculaire auxdites au moins deux électrodes à sec (EL1, EL2).
